# EUROPÄISCHE PATENTSCHRIFT

(11) **EP 0 827 002 B1**
(45) Veröffentlichungstag und Bekanntmachung des Hinweises auf die Patenterteilung: **09.06.2004**
(21) Anmeldenummer: 97119034.3
(22) Anmeldetag: 09.04.1995
(51) Int. Cl.: G02B 21/00, G01B 9/04

(54) **Operationsmikroskopsystem mit Datenaufbereitungseinheit**
Surgical microscope system with data processing unit
System d' un microscope chirurgical aved un processeur des données

(30) Priorität: 11.04.1994 CH 108894; 11.04.1994 CH 108994; 11.04.1994 CH 109094; 11.04.1994 CH 109194; 11.04.1994 CH 109294
(43) Veröffentlichungstag der Anmeldung: 04.03.1998
(62) Teilanmeldung aus: 95914354.6
(73) Patentinhaber: Leica Mikroskopie Systeme AG, 9435 Heerbrugg (CH)
(72) Erfinder: Spink, Roger, 9442 Berneck (CH); Braunecker, Bernhard, 9445 Rebstein (CH); Mayer, Thomas, 6845 Hohenems (AT); Zimmer, Klaus-Peter, 9435 Heerbrugg (CH); Rogers, John Rice, Sierra Madre, CA 91024 (US)
(74) Vertreter: Stamer, Harald

(56) Entgegenhaltungen:
- EP-A- 0 164 680
- EP-A- 0 456 103
- WO-A-88/07312
- WO-A-93/16631
- DE-A- 4 134 481
- DE-A- 4 202 505
- NL-A- 9 000 622
- US-A- 4 122 518
- US-A- 4 638 471
- US-A- 4 672 559
- US-A- 4 686 639
- US-A- 5 098 426
- US-A- 5 108 174
- US-A- 5 249 581
- US-A- 5 279 309

## Beschreibung

Unter Datenaufbereitungseinheit ist insbesondere jede komplette oder modulare Einrichtung zu verstehen, die im Zusammenhang mit einem Mikroskop bzw. im Zusammenhang mit an einem Mikroskop durchgeführten Untersuchungen verwendet wird, um Daten zu verarbeiten, die für die Bedienung oder für die Erkenntnisse aus dem Mikroskop oder für die Arbeit unter dem Mikroskop von Bedeutung sind. Als Datenverarbeitungseinheit sind beispielsweise Mikroprozessoren, Computer oder Workstations zu verstehen, mit deren Hilfe beispielsweise Daten aus dem Mikroskop erfasst werden, Positionsdaten des Mikroskops erfasst und eventuell weiterverarbeitet oder - geleitet werden, oder mit deren Hilfe das Mikroskop beispielsweise selbst angesteuert wird.

Unter einem Display im Sinne der Erfindung sind beispielsweise Bildschirme, Kathodenstrahlröhren etc. zu verstehen, auf denen Informationen für einen Benutzer erscheinen. Solche Displays können sowohl ausserhalb des Mikroskops, z.B. als Computerbildschirm angeordnet sein, oder auch als kleine Displays ausgebildet sein, die so mit dem optischen Strahlengang des Mikroskops verbunden sind, dass ein Anwender sowohl eine optische Wahrnehmung aus dem optischen Strahlengang als auch gleichzeitig (überlagert) eine optische Wahrnehmung aus dem Display erhält.

In besonderen Fällen kann im weitesten unter Display auch eine akustische Informationsvorrichtung verstanden werden.

Videomikroskope im Sinne der Erfindung sind Mikroskope mit wenigstens einem lichtoptischen Strahlengang und wenigstens einer Bildaufnahmevorrichtung zur Aufnahme und Darstellung des über den Strahlengang gesehenen Bildes auf einem Display. Zu einer sehr häufigen Art von Videomikroskopen sind in der letzten Zeit Videostereomikroskope geworden, bei denen zwei parallele Strahlengänge vorgesehen sind und auf dem Display ein 3-D-Bild darstellbar ist. Videomikroskope werden häufig als Operationsmikroskope benutzt; wobei im Rahmen der Erfindung auch alle anderen Mikroskope und Endoskope liegen, die oben beschriebene Vorrichtungen aufweisen.

Vor allem bei Operationsmikroskopen und insbesondere während einer Operation fallen eine Menge von Informationen an, die für den Chirurgen von grosser Bedeutung sein können. Dies sind beispielsweise Informationen über bestimmte Parameter des Mikroskops. Dies sind aber auch Informationen über die Lage des beobachteten Operationsfeldes, Informationen über den Patienten bzw. dessen Gesundheitszustand bzw. dessen Parameter wie Puls, Blutdruck, Sauerstoffgehalt des Blutes usw. und beispielsweise auch Vergleichsdaten aus älteren, über Video aufgezeichneten Mikroskopaufnahmen oder Aufzeichnungen aus anderen Aufnahmeverfahren wie Röntgen-, Ultraschall-, Positronenstrahl- oder MRI-Aufnahmen.

Zusätzlich zu diesen Informationen fallen auch eine Menge von Steuerdaten an, die beispielsweise vom Chirurgen willkürlich über Steuerorgane wie Computermouse, Fussschalter usw. an die Datenverarbeitung bzw. an Steuerorgane für das Mikroskop abgegeben werden, um dieses nach Bedarf zu steuern, z.B. zu fokussieren.

Vor allem bei jenen Anwendungen, wo Bilder überlagert werden, sei es optisch oder optoelektronisch, beispielsweise durch Displays, die über Strahlenteiler in den Okularstrahlengang eingespiegelt werden, oder rein elektronisch, beispielsweise durch Bildverarbeitung und gleichzeitige Darstellung von überlagerten Bildern auf einem Display tritt im Zusammenhang mit der elektronischen Datenverarbeitung ein Problem auf: Während Operationen ist der Chirurg einerseits auf eine Echtzeitdarstellung und andererseits auf ein rasches Reagieren von Steuerorganen des Mikroskopes und auf eine exakte Positionierung des Mikroskopes bzw. dessen Sehfeldes angewiesen.

Dies bedeutet bei herkömmlichen Aufbauten, dass enorm grosse Rechnerleistungen von der Datenverarbeitungsanlage gefordert werden. Diese Rechnerleistung wird verbraucht durch das Aufnehmen und Abspeichern von Daten, Umrechnen derselben in andere Daten, Umwandeln von Daten von analogen Werten in digitale Werte oder umgekehrt, gegebenenfalls Vergleichen von abgespeicherten oder parallel aufgenommenen Daten mit aufgenommenen Daten und Abgeben von Daten an Steuerorgane, Displays, Anzeigen, Datennetze usw. Gleichzeitig müssen in der elektronischen Datenverarbeitung auch vielfältige Softwareprogramme geladen sein, die eine Vernetzung untereinander erforderlich machen, was häufig kompliziert und entsprechend kostenaufwendig ist.

In der US-A-5073857 ist eine Methode und eine Vorrichtung für eine Zellenanalyse beschrieben, die unter anderem eine Videokamera, einen Bildgenerator, einen Mikroprozessor, eine Zählvorrichtung, eine Vergleichseinrichtung, einen Digital-Analogkonverter, eine Steuereinrichtung für das Mikroskop und einen Computer aufweist. Der Computer ist dabei nur als verlängertes Eingabe/Ausgabegerät für den Mikroprozessor vorgesehen und diesem ist ein Programmspeicher zugeordnet, so dass er für sich allein eher als eigentlicher Computer anzusehen ist, der wieder die erwähnt hohe Rechnerleistung erfordert, um ein Arbeiten in Echtzeit zu ermöglichen.

US-A-4 672 559 beschreibt ein Mikroskop mit einer Vorrichtung zum Einblenden von Bilddaten. Die Mikroskopanordung beinhaltet einen Computer und einen Bildprozessor.

Demgegenüber stellt sich der Erfindung die Aufgabe, eine Anordnung zur Datenverarbeitung bzw. ein Mikroskop mit einer solchen Anordnung zu schaffen, bei der bzw. bei dem mit schnellsten Verarbeitungszeiten in Echtzeit gearbeitet werden kann und bei der bzw. bei dem die erforderliche Rechnerleistung in der Datenverarbeitungsanlage selbst gering gehalten werden kann. Auch softwaremässig soll ein kompakter Aufbau mit einfachen Erweiterungsmöglichkeiten gegeben sein.

Gelöst wird diese Aufgabe durch die Anwendung der Merkmale des Anspruches 1. Die Erfindung als Ganzes stellt dabei eine mit dem Mensch vergleichbare Anordnung dar: Sinnesorgane und Extremitäten sind über das Rückenmark, das selbst bestimmte Funktionen erfüllt, mit dem Gehirn (Computer) verbunden.

Durch die erfindungsgemässe Anordnung werden alle interessierenden Daten kontinuierlich und selbsttätig so aufbereitet, dass der angeschlossene Computer (in vielen Fällen eine Workstation) Zugriff auf optimale und vereinheitlichte Datenformate hat, die unabhängig von seiner Rechnerleistung ermittelt und aufbereitet werden. Der Computer muss nicht mehr wie bisher alle Rechenoperationen durchführen; seine EDV-Leistung kann auf die jeweils zur Berechnung in seinem Arbeitsspeicher vorgesehenen Rechenoperationen beschränkt werden, wodurch er selbst optimal ausgelegt werden kann. Das von den Anwendem geforderte Echtzeit-Verhalten ist durch die Erfindung einfach zu erreichen.

Besondere Ausführungsformen der Anordnung sind in den abhängigen Patentansprüchen beschrieben bzw. unter Schutz gestellt. Sie erleichtern bei ihrer Anwendung im Operationsmikroskopiebereich vor allem die jeweils anwenderspezifische Arbeitsweise.

Die erfindungsgemässe Anordnung führt somit zu kompakteren, schnelleren und anwendersichereren Mikroskopen bzw. Arbeitsweisen mit Mikroskopen, was vor allem im Bereich der Mikrochirurgie von enormem Vorteil ist.

Die folgenden Patentanmeldungen , die andere Aspekte eines erfinderischen neuen Mikroskopes unter Schutz stellen können bevorzugt auch mit der vorliegend beschriebenen Anordnung ausgerüstet sein: Es sind dies die Patentanmeldungen: WO-A-95 27 226, WO-A-95 27 917 und WO-A-95 27 918.

Weitere Details und Ausführungen der Erfindung ergeben sich aus der Zeichnung. Die dort dargestellten Figuren zeigen:
- Fig.1: ein Prinzipschaltbild eines Aufbaus gemäss Anspruch 6 und
- Fig. 2: ein detailierteres, sich selbst erklärendes Prinzipschaltbild eines ähnlichen Aufbaus.

Die Figuren werden zusammenhängend beschrieben. Gleiche Bezugszeichen bedeuten gleiche Bauteile. Gleiche Bezugszeichen mit unterschiedlichen Indizes bedeuten ähnliche bzw. funktionsähnliche Bauteile. Die Erfindung ist auf die dargestellten Ausführungsbeispiele nicht eingeschränkt. Vor allem in Kombination mit den Lehren der oben angeführten PCT-Patentanmeldungen lassen sich noch beliebige Varianten darstellen. Sie alle fallen unter den Offenbarungsinhalt dieser Anmeldung. Die angefügte Bezugszeichenliste ist deshalb dementsprechend fortlaufend geführt.

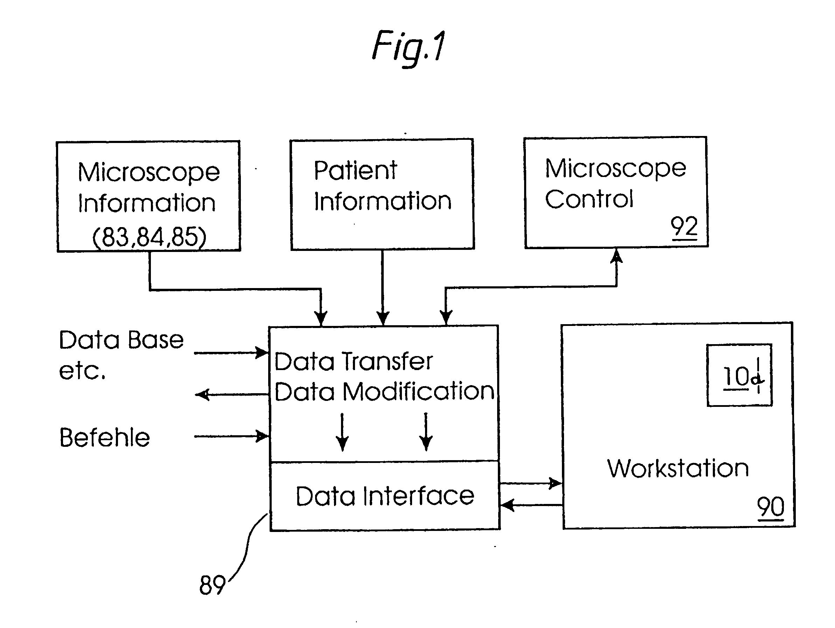

Das Prinzip der Erfindung wird in Fig.1 verdeutlicht: Mehrere periphere Geräte werden an einer Datenaufbereitungseinheit 89 zusammengeführt um dort Daten für die Weiterverarbeitung in einem Computer 90 aufzubereiten. Die Datenaufbereitungseinheit 89 wandelt bei Bedarf allerdings auch Daten aus dem Computer 90 in solche Datenformate um, dass diese unmittelbar und direkt in den peripheren Geräten eingesetzt werden können. Bestimmte Daten, deren Bearbeitung in der Workstation nicht erforderlich ist, können nach ihrer Aufarbeitung (Umrechnung, Formatangleichung, Verknüpfung mit anderen Daten) gegebenenfalls auch unmittelbar für andere periphere Geräte zur Verfügung gestellt werden.

Als Beispiel sei eine gewünschte Vergrösserung angeführt, die vom Anwender per Befehl an die Anordnung bzw. an die Datenaufbereitungseinheit 89 gemeldet wird. Der Befehl, der beispielsweise ein Zahlenwert ist, wird mit einem gemessenen Wert der Vergrösserung aus der Vorrichtung 85 verglichen, wobei dies zunächst z.B. ein analoger Spannungswert oder ein digitaler Ortsvektorenwert ist, welche Werte durch die Datenaufbereitungseinheit 89 zunächst automatisch umgerechnet werden, um einen Vergleich zwischen den beiden Soll- und Ist-Daten zu ermöglichen. Zeigt der Vergleich einen Unterschied, so wird beispielsweise ein entsprechender Steuerwert für die Mikroskopsteuerorgane 92 erzeugt, um eine entsprechende Regelung nach Art eines Regelkreises zu ermöglichen.

Auch können beispielsweise Patienteninformationsdaten in anschaulicher Form auf einem Display zur Ansicht gebracht werden, wobei auf demselben Display gleichzeitig entsprechende Vergleichsdaten aus einer Datenbank dargestellt werden. Auch solche Daten können im Rahmen der Erfindung beispielsweise aufbereitet werden, ohne die Rechnerleistung des Computers 90 in Anspruch zu nehmen. Diese steht daher für aufwendigere Rechenmanipulationen zur Verfügung, z.B. für das Umwandeln von MRI-Bilddaten zur Vergrösserung oder Verkleinerung des MRI-Bildes entsprechend den eingestellten Vergrösserungswerten am Mikroskopstrahlengang 60 und zur bildverarbeitenden Überlagerung des MRI-Bildes dem durch den Mikroskopstrahlengang 60 gesehenen und z.B. mittels Videokamera 9 aufgezeichneten Bild, wobei die vollzogene Überlagerung für den Anwender an einem Display 10a und/oder 10b dargestellt wird.

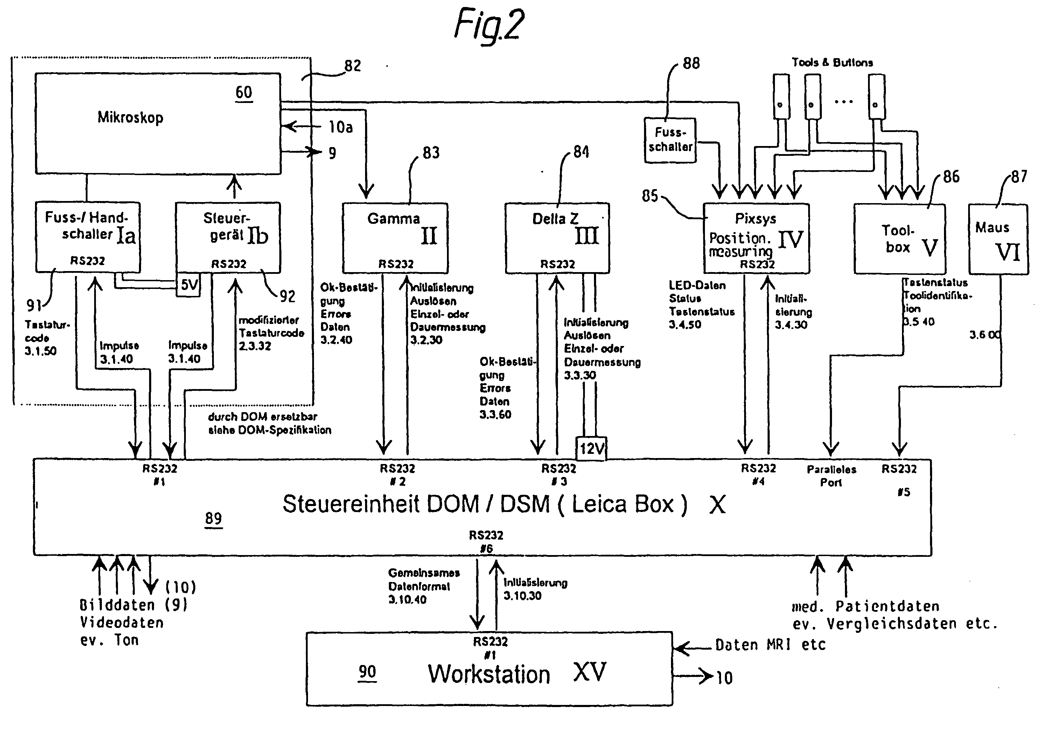

Die Angaben gemäss Fig.2 sind für einen Fachmann selbsterklärend.

Ein Mikroskop 82 nach Anspruch 6 kann mit verschiedenen peripheren Geräten, die in den oben genannten Patentanmeldungen gezeigt sind, wie Vorrichtung 83 zur automatischen, elektronisch unterstützten Vergrösserungsmessung durch die Mikroskopoptik 60, Vorrichtung 84 zur automatischen, elektronisch unterstützten Distanzmessung zwischen betrachtetem Objekt und Mikroskop 82, Vorrichtung 85 zur automatischen und elektronisch unterstützten Positionsbestimmung des Mikroskops 82 im Raum, Vorrichtung 88 zur Ansteuerung von Positionsänderungsantrieben des Mikroskops 82, Datenbank, Videokamera, Vorrichtung für das Erfassen von Patientendaten wie z.B. Name, Puls, Blutdruck, Sauerstoffgehalt des Blutes usw. verbunden werden und zugleich auch mit der Datenaufbereitungseinheit 89 verbunden werden, die Daten - bevor sie in eine angeschlossene Workstation gehen oder wenn sie daraus kommen - formatrichtig aufarbeitet oder umrechnet, um einen Echtzeit Betrieb mit relativ geringen Rechnerleistungen in der Workstation zu ermöglichen.

### Bezugszeichenliste

Diese Bezugszeichenliste enthält auch Bezugszeichen von Figuren, die in den oben erwähnten Anmeldungen beinhaltet sind, da diese, bzw. die durch diese Bezugszeichen angeführten Merkmale und deren entsprechenden Beschreibungs- und Zeichnungsteile, wie erwähnt als im Rahmen dieser Erfindung liegend zu Kombinationszwecken mitgeoffenbart gelten. Insbesondere betrifft dies die Mikroskope mit speziellen Strahlengängen und Strahlenteilem und die Vorrichtungen zum Messen der Vergrösserung und des Abstandes vom Mikroskop zum Objekt.
- 1: erster Strahlengang; a,b
- 2: zweiter Strahlengang (geometrisch übereinander gelegte erste Strahlengänge); a,b
- 3: mechanooptisches Schaltelement
3a-c undurchlässige und vorzugsweise verspiegelte Blende
3d LCD-Shutter-Element
3e mikromechanische Lamellenspiegelkonstruktion
3f LCD Wechselshutterelement
- 4: Strahlenteiler
4a,b Strahlenteiler
4c Strahlenteiler für Messstrahlausblendung 4c1, 4c2
- 5: Scheibe
5a halbkreisförmige Fläche -
5b Restfläche der Scheibe 5
5c 5d Kreissegmentflächen
- 6: Achse für Scheibe
- 7: Mittelachse
7a,b Mittelachse
- 8: Hauptobjektiv
8a Hauptobjektiv
8b Hauptobjektiv mit 8a vertauschbar (unterschiedliche Brennweiten)
- 9: elektronische Bildaufnahmevorrichtung
- 10: Display
10a Display
- 11: Spiegel; a,b
- 12: Verstelleinrichtung; a-c
- 13: Zoom
- 14: Motor; a,b
- 15: Reziprokantrieb
- 16: Zuleitung
- 17: Lichtquelle
- 18: Okular
- 19: Umlenkspiegel
- 20: Schubstange
- 21: starrer Spiegel
- 22: Objekt
22a Objektdetail
- 23: Planplatte; a-d,a',b'
- 24: Schwenkantrieb
- 25: Gestänge
- 30: Lamellenspiegel von 3e
- 31: Tubuslinse
- 32: Einblendelement
32a Strahlenteiler
32b Spiegel
32c zweites Einblendelement
33 Vergrösserungsoptik
- 34: Pfeile
- 35: weiterer Spiegel
- 36: Stellantrieb
- 37: Balken
- 38: Umlenkspiegel; a,b
- 39: Retroprisma
- 40: Ausgleichsgewicht
- 41: Trägerplatte a,b,c: prismatische mit integriertem Spiege
- 42: Farbfilter, a-f
- 43: Intervallschalter
- 44: Mikroprozessor
- 45: Messarray a
- 46: Referenzarray a
- 47: Modul für Bilddatenübertragung
- 48: Fremdbilddateninput
- 49: Stellmotor für Zoom 13; a,b
- 50: Verbindungsleitungen a-g
- 51: Vergrösserungsanzeige a-c
- 52: Kurvenscheibe
- 53: Kopplung
53a zwischen Stellmotor 49b und Zoom 13 bzw. zwischen 49 und 52
53b zwischen Kurvenscheibe 52 und Vergrösserungsanzeige 51b
- 54: mechanischer Abgriff
- 55: Zeiger; a,b
- 56: Laser
- 57: Messstrahl a-c,c1
- 58: Referenzstrahl
- 59: Pfeile für Verschiebbarkeit des Einblendelementes 32
- 60: Mikroskopstrahlengang a-e
- 61: erstes Umlenkelement a
- 62: Fokussierelement a,b
- 63: Lichtleiterendstück a,b
- 64: Lichtquelle a
- 65: zweites Umlenkelement
- 66: Sensor
- 67: Distanzbereich a
- 68: Verbindungsleitung
- 69: Distanzmesssystem
- 70: Verbindung
- 71: Vergrösserungsmesseinheit
- 72: Positionsbestimmungssystem a,b
- 73: Interferometer
- 74: halbdurchlässiger Spiegel
- 75: Reflektor
- 76: Detektor
- 77: elektromechanisches Verstellelement
- 78: Interferometersteuerung
- 79: Gitter
- 80: Detektor-CCD
- 81: Stufen
- 82: Mikroskop
- 83: Anordnung zur Vergrösserungsmessung des Mikroskopes
- 84: Anordnung zur Entfernungsmessung Objekt/Mikroskop
- 85: Positionsmessystem zur Bestimmung der Absolutlage des Mikroskopes im Raum um daraus nach Kenntnis der Entfernung Objekt/Mikroskop auch auf die Lage des Sehfeldes am Objekt schliessen zu können
- 86: Toolbox für verschiedene Anwenderprogramme
- 87: Befehlsteuerorgan (Computermouse)
- 88: Befehlsteuerorgan zur Bewegungssteuerung des Mikroskopes (z.B. Fussschalter)
- 89: Datenaufbereitungseinheit
- 90: Computer (Workstation)
- 91: Steuerschalter für Mikroskop
- 92: elektromechanische Steuereinheit für Mikroskop (Zoom, Fokus etc.)
- 93: Leuchtdioden; a-c
- 94: Glasfasern; a-c
- 95: Enden der Glasfasern; a-c
- 96: IR-Rezeptoren; a-c
- 97: Mikroskopständer
- 98: Rückkopplung
- 99: Zuleitungen; a-c
- 100: Reflektoren mit spezieller Oberfläche
- b: Abstand der Messstrahlen 57a und 57b
- b': Abstand der Messstrahlen 57a und 57b am Messarray
- d1,2: Stereobasis

## Patentansprüche

1. Operationsmikroskop-System mit einem Operationsmikroskop (82), einem Computer (90) und einer Datenaufbereitungseinheit (89), t etztere als Dateninterface zur Echtzeitverarbeitung von Daten für das Operationsmikroskop (82) und den Computer (90) für das weitere Verbinden mit einem Display (10) und/oder mit peripheren Geräten mit vereinheitlichten Datenformaten,
wobei die Datenaufbereitungseinheit (89) Ein- und Ausgänge aufweist, an denen gleichzeitig Datensätze mit unterschiedlichen Datenformaten oder umzurechnenden Datenwerten aus Mess-, Steuer-, Video-, Ton-, Vergleichs- und Patientendatenbanken anlegbar sind,
wobei die Datenaufbereitungseinheit (89) mit Software versehen ist, die eingehende Daten nach Bedarf umwandelt oder umrechnet, sodass einheitliche Datenformate und/oder aufbereitete Daten direkt oder in bestimmter Reihenfolge abrufbar werden,
und wobei die Datenaufbereitungseinheit (89) mit dem Computer (90) derart verbunden ist, dass dieser im Betriebszustand die von seiner Software benötigten Daten direkt abrufen und weiterverarbeiten kann und von der Datenaufbereitungseinheit (89) abgehende Daten für die direkte Anwendung in den peripheren, ebenfalls an der Datenaufbereitungseinheit (89) anschließbaren Geräten (83, 84, 85, 86, 87) umgewandelt bzw. umgerechnet werden.

2. System nach Anspruch 1, **dadurch gekennzeichnet, dass die** Datenaufbereitungseinheit (89) über serielle und parallele Datenein- und -ausgänge verfügt.

3. System nach Anspruch 1 oder 2, **dadurch gekennzeichnet, dass die** Software der Datenaufbereitungseinheit (89) das automatische Überlagern von Informationsdaten für den Anwender mit Bilddaten, die dem Anwender über ein Display (10) sichtbar gemacht werden, erlaubt, ohne dazu Rechenoperationen im Computer (90) zu erfordern.

4. Anordnung System nach einem der Ansprüche 1-3, **dadurch gekennzeichnet, dass** die Datenaufbereitungseinheit (89) mit wenigstens einem der folgenden peripheren Geräte verbunden ist:
Vorrichtung (83) zur automatischen, elektronisch unterstützten Vergrösserungsmessung durch die Mikroskopoptik (60), Vorrichtung (84) zur automatischen, elektronisch unterstützten Distanzmessung zwischen betrachtetem Objekt und Mikroskop (82), Vorrichtung (85) zur automatischen und elektronisch unterstützten Positionsbestimmung des Mikroskops (82) im Raum, Vorrichtung (88) zur Ansteuerung von Positionsänderungsantrieben des Mikroskops (82), Datenbank, Videokamera, Vorrichtung für das Erfassen von Patientendaten wie z.B. Name, Puls, Blutdruck, Sauerstoffgehalt des Blutes usw.

5. System nach einem der Ansprüche 1-4, **dadurch gekennzeichnet, dass** Datenaufbereitungseinheit (89) mit allen im Anspruch 25 aufgelisteten peripheren Geräten verbunden ist.

6. System, nach einem der Ansprüche 1-5 mit elektronischen Messvorrichtungen (83-85) und elektromechanischen Stellorganen (91,92) und einem Interface zu einem Computer (90), **dadurch gekennzeichnet, dass** das Interface eine Datenaufbereitungseinheit (89) umfasst, die mit Software ausgerüstet ist, um eingehende Daten in für die Weiterverarbeitung durch den Computer (90) optimale Datenformate bzw. Datenwerte umzuwandeln, so dass im Betriebszustand der Computer (90) auf von seiner Software benötigte Daten direkten und unmittelbaren Zugriff hat und/oder so dass vom Computer (90) gelieferte Daten für die unmittelbare Anwendung in den elektronischen Messvorrichtungen oder elektromechanischen Stellorganen verfügbar gemacht werden.

7. System nach Anspruch 6, **dadurch gekennzeichnet, dass** es wenigstens mit zwei der folgenden Geräte ausgerüstet ist, deren Datenausund -eingänge mit der Datenaufbereitungseinheit (89) verbunden sind:
Vorrichtung (83) zur automatischen, elektronisch unterstützten Vergrösserungsmessung durch die Mikroskopoptik (60), Vorrichtung (84) zur automatischen, elektronisch unterstützten Distanzmessung zwischen betrachtetem Objekt und Mikroskop (82), Vorrichtung (85) zur automatischen und elektronisch unterstützten Positionsbestimmung des Mikroskops (82) im Raum, Vorrichtung (88) zur Ansteuerung von Positionsänderungsantrieben des Mikroskops (82), Datenbank, Videokamera, Vorrichtung für das Erfassen von Patientendaten wie z.B. Name, Puls, Blutdruck, Sauerstoffgehalt des Blutes usw.

8. System nach Anspruch 6 oder 7, **dadurch gekennzeichnet, dass** es über die Datenaufbereitungseinheit (89) mit allen Geräten nach Anspruch 7 verbunden ist.

9. System nach einem der Ansprüche 6-8, **dadurch gekennzeichnet, dass** als Mikroskop (82) ein Videostereomikroskop eingesetzt ist.

10. System nach Anspruch 9 mit einem Display (10a) dessen Bild in den Mikroskopstrahlengang (60) einblendbar ist.

## Claims

1. Surgical microscope system having a surgical microscope (82), a computer (90) and a data conditioning unit (89), the latter as a data interface for real time processing of data for the surgical microscope (82) and the computer (90) for further connection to a display (10), and/or having peripheral devices with standarized data formats, the data conditioning unit (89) having inputs and outputs to which it is possible to apply simultaneously data records with different data formats or data values which are to be converted and are composed of measurement, control, video, audio, comparative and patient databases,
the data conditioning unit (89) being provided with software which transforms or converts incoming data according to requirements such that uniform data formats and/or conditioned data can be retrieved directly and in a specific sequence, and it being possible to connect the data conditioning unit (89) to the computer (90) in such a way that in the operating state the said computer can retrieve the data required by its software directly and can process them further, and data outgoing from the data conditioning unit (89) is transformed or converted for direct use in the peripheral devices ( 83, 84, 85, 86, 87) which can likewise be connected to the data conditioning unit (89).

2. System according to Claim 1, **characterized in that** the data conditioning unit (89) has serial and parallel data inputs and data outputs.

3. System according to Claim 1 or 2, **characterized in that** the software of the data conditioning unit (89) permits the automatic superposition of information data for the user with image data which are visualized for the user via a display (10) without this requiring arithmetic operations in the computer (90).

4. System according to one of Claims 1-3, **characterized in that** the data conditioning unit (89) is connected to at least one of the following peripheral devices:
Apparatus ( 83) for automatic, electronically aided magnification measurement by the microscope optical system (60), apparatus (84) for automatic, electronically aided distance measurement between the object under view and the microscope (82), apparatus (85) for automatic and electronically aided determination of the position of the microscope (82) in space, apparatus (88) for driving position-changing drives of the microscope (82), database, video camera, and apparatus for acquiring patient data such as, for example, name, pulse, blood pressure, oxygen content of the blood etc.

5. System according to one of Claims 1-4, **characterized in that** the data conditioning unit (89) is connected to all the peripheral devices listed in Claim 4.

6. System according to one of Claims 1-5, having el ectronic measuring apparatuses (83-85) and electromechanical regulating units (91, 92) and an interface to a computer (90), **characterized in that** the interface comprises a data conditioning unit ( 89) which is equipped with software in order to convert incoming data into data formats or data values which are optimum for further processing by the computer (90), such that in the operating state the computer (90) has direct and immediate access to data required by its software, and/or such that data supplied by the computer (90) are made available for immediate application in the electronic measuring apparatuses or electromechanical regulating units.

7. System according to Claim 6, **characterized in that** it is equipped with at least two of the following devices whose data outputs and inputs are connected to the data conditioning unit (89):
Apparatus (83) for automatic, electronically aided magnification measurement by the microscope optical system (60), apparatus (84) for automatic, electronically aided distance measurement between the object under view and the microscope (82), apparatus ( 85) for automatic and el ectronicall y aided determination of the position of the microscope (82) in space, apparatus (88) for driving position-changing drives of the microscope (82), database, video camera, and apparatus for acquiring patient data such as, for example, name, pulse, blood pressure, oxygen content of the blood etc.

8. System according to Claim 6 or 7, **characterized in that** it is connected via the data conditioning unit (89) to all the devices according to Claim 7.

9. System according to one of Claims 6-8, **characterized in that** a video stereomicroscope is used as the microscope (82).

10. System according to Claim 9, having a display (10a) whose image can be inserted into the microscope beam path (60).

## Revendications

1. Système de microscope chirurgical avec un microscope chirurgical (82), un ordinateur (90) et une unité de traitement des données (89), cette dernière constituant l'interface des données vers le traitement en temps réel de données pour le microscope chirurgical (82) et l'ordinateur (90), pour la liaison ensuite avec un afficheur (10) et/ou avec des équipements périphériques avec des formats de données uniformisés,
l'unité de traitement des données (89) présentant des entrées et des sorties auxquelles on peut appliquer simultanément des trains de données de formats de données divers ou des valeurs de données à convertir provenant de banques de données de mesures, de commande, de vidéo, de son, de comparaison et de patients,
l'unité de traitement des données (89) étant pourvue de logiciel qui transforme ou convertit les données d'entrée selon les besoins de manière à pouvoir appeler directement ou selon une séquence définie, des formats de données et/ou des données élaborées uniformes,
et l'unité de traitement des données (89) étant reliée à l'ordinateur (90) de manière à lui permettre, en état de fonctionnement, d'appeler directement et de poursuivre le traitement des données nécessaires à son logiciel et à transformer, respectivement à convertir, les données quittant l'unité de traitement des données (89) pour l'application directe dans les équipements périphériques (83, 84, 85, 86, 87) pouvant également se raccorder à l'unité de traitement des données (89).

2. Système selon la revendication 1, **caractérisé en ce que** l'unité de traitement des données (89) dispose d'entrées et de sorties de données série et parallèles.

3. Système selon la revendication 1 ou 2, **caractérisé en ce que** le logiciel de l'unité de traitement des données (89) permet la superposition automatique de données d'information pour l'utilisateur avec des données d'image qui sont rendues visibles pour l'utilisateur au moyen d'un afficheur (10) sans nécessiter pour cela des opérations de calcul dans l'ordinateur (90).

4. Système selon une des revendications 1 - 3, **caractérisé en ce que** l'unité de traitement des données (89) est reliée à un au moins des équipements périphériques suivants :
dispositif (83) pour la mesure automatique aidée par l'électronique du grandissement par l'optique du microscope (60), dispositif (84) pour la mesure automatique aidée par l'électronique de la distance entre l'objet observé et le microscope (82), dispositif (85) pour la détermination automatique aidée par l'électronique de la position dans l'espace du microscope (82), dispositif (88) pour la commande d'entraînements de modification de la position du microscope (82), banque de données, caméra vidéo, dispositif pour la saisie de données de patients comme par exemple le nom, le pouls, la pression sanguine, le taux d'oxygène du sang etc.

5. Système selon une des revendications 1 - 4, **caractérisé en ce que** l'unité de traitement des données (89) est reliée à tous les équipements périphériques énumérés dans la revendication 4.

6. Système selon une des revendications 1 - 5 avec des dispositifs de mesure électroniques (83 - 85) avec des organes de réglage électromécaniques (91, 92) et une interface avec un ordinateur (90), **caractérisé en ce que** l'interface comprend une unité de traitement des données (89) pourvue de logiciel pour transformer les données d'entrée en des formats de données ou selon le cas des valeurs de données optimaux pour le traitement ultérieur par l'ordinateur (90) de telle sorte qu'en état de fonctionnement, l'ordinateur (90) ait un accès direct et immédiat aux données nécessaires à son logiciel et/ou de telle sorte que les données fournies par l'ordinateur (90) soient mises à disposition pour l'utilisation immédiate dans les dispositifs de mesure électroniques ou dans les organes de réglage électromécaniques.

7. Système selon la revendication 6, **caractérisé en ce qu'**il est pourvu d'au moins deux des équipements suivants, dont les sorties et les entrées de données sont reliées à l'unité de traitement des données (89) : dispositif (83) pour la mesure automatique aidée par l'électronique du grandissement par l'optique du microscope (60), dispositif (84) pour la mesure automatique aidée par l'électronique de la distance entre l'objet observé et le microscope (82), dispositif (85) pour la détermination automatique aidée par l'électronique de la position dans l'espace du microscope (82), dispositif (88) pour la commande d'entraînements de modification de la position du microscope (82), banque de données, caméra vidéo, dispositif pour la saisie de données de patients comme par exemple le nom, le pouls, la pression sanguine, le taux d'oxygène du sang etc.

8. Système selon la revendication 6 ou 7, **caractérisé en ce qu'** il est relié par l'intermédiaire de l'unité de traitement des données (89) à tous les équipements selon la revendication 7.

9. Système selon une des revendications 6 - 8, **caractérisé en ce qu'**un microscope stéréoscopique vidéo est mis en oeuvre comme microscope (82).

10. Système selon la revendication 9 avec un afficheur (10a) dont l'image peut être incrustée dans le trajet des rayons du microscope (60).
